# EUROPEAN PATENT APPLICATION

(11) **EP 1 911 470 A1**
(43) Date of publication of application: **16.04.2008**
(21) Application number: 07118192.9
(22) Date of filing: 10.10.2007
(51) Int. Cl.: A61L 27/54

(54) **Antimicrobial medical implement containing silver ions**

(30) Priority: 12.10.2006 JP 2006279088
(71) Applicant: Covidien AG, 8212 Neuhausen am Rheinfall (CH)
(72) Inventor: Amano, Kenichi, Fukuroi-Shi Shizuoka 437-0004 (JP); Akaike, Yoshimi, Fukuroi-Shi Shizuoka 437-0004 (JP)
(74) Representative: Tomlinson, Edward James

(57) **Abstract**

The present disclosure provides medical implements having films fixed to at least a portion of their surface In embodiments, a medical implement of the present disclosure may include a substrate including a synthetic resin, a film including a methyl vinyl ether-maleic anhydride copolymer in combination with a polyether block amide fixed to at least a portion of a surface of the substrate, and a silver ion bound to the film. Methods for forming such medical implements are also provided

## Description

### CROSS-REFERENCE TO RELATED APPLICATIONS

This application claims the benefit of and priority to Japanese Patent Application No 2006-279088 filed on October 12, 2006, the entire disclosure of which is hereby incorporated by reference herein.

### TECHNICAL FIELD

The present disclosure provides medical implements and methods for producing such implements In embodiments, the present disclosure provides medical implements possessing films with antimicrobial properties, and optionally other beneficial properties, formed on the surface of substrates made of synthetic resins, and medical implement production methods

### BACKGROUND

It may be desirable for catheters that are inserted in the body, for example, when used in central venous nutrition or artificial dialysis, to have added antimicrobial properties to prevent the entry of bacteria from the insertion site For example, Kokai Patent Application No. Hei 11 [1999]-290449, describes catheters in which silver compounds have been introduced on the surface as antimicrobial agents by immersing the catheters in aqueous silver solutions after compounds capable of binding silver ions have been adsorbed onto them.

In addition to the above antimicrobial properties, medical implements that are inserted in the body may be endowed with other properties. For example, to prevent injury to the mucosa and perform a smooth insertion when these kinds of medical implements are inserted in the body, lubricant properties may be required For medical implements that contact the blood, such as catheters that remain in blood vessels, antithrombotic properties may be required to prevent formation of blood clots due to blood coagulation

### SUMMARY

The present disclosure provides medical implements having films on at least a portion thereof that possess antimicrobial properties and which can possess other desirable properties, and methods for manufacturing such medical implements

In embodiments, medical implements may include a substrate made of synthetic resin and a film on at least a portion of the surface of the substrate In embodiments, the film may include a methyl vinyl ether-maleic anhydride copolymer with bound silver ions

Since silver may be bound to the methyl vinyl ether-maleic anhydride copolymer as a main component of the film formed on the surface of the synthetic resin substrate, this silver exhibits antimicrobial effects Moreover, since the methyl vinyl ether-maleic anhydride copolymer is capable of binding enzymes with activities that lyse thrombin (for example, fibrin lumps or fibrin) such as urokinase, they can be made into medical implements with antimicrobial and antithrombotic properties by binding antithrombotic substances such as urokinase in addition to silver. Furthermore, with alkali treatments, methyl vinyl ether-maleic anhydride copolymers form salts with bound alkali metals and may exhibit lubricant properties when wet, so they can also be made into medical implements having lubricant properties.

As noted above, in embodiments, in addition to silver ions, antithrombotic substances may be bound to a film on a medical implement of the present disclosure to prevent formation of thrombi or to solubilize clots themselves Furthermore, it may be desirable that salts binding alkali metals such as sodium may be formed by alkali treatment.

In embodiments, the methyl vinyl ether-maleic anhydride copolymer may be fixed to the substrate surface via a polyether block amide Because the polyether block amide serves as a binder and firmly fixes the methyl vinyl ether-maleic anhydride copolymer to the synthetic resin substrate, peeling of the film from the substrate is prevented, and thus the properties attributed to the film, in embodiments antimicrobial properties, lubricant properties and antithrombotic properties, can be maintained for a long time.

The present disclosure also provides medical implement production methods that include a process wherein a mixture of solution possessing solubilized methyl vinyl ether-maleic anhydride copolymer and a solution with solubilized polyether block amide is applied on the surface of a substrate made of synthetic resin, and a film is formed on the surface of the substrate. A second solution with a solubilized silver compound may be applied on the surface of the substrate on which the above film is formed, whereby silver ions become bound to the film

Because methyl vinyl ether-maleic anhydride copolymer and polyether block amide are applied on the surface of the synthetic resin substrate, a film having methyl vinyl ether-maleic anhydride copolymer as the main component is formed on the substrate surface This film is firmly fixed to the substrate surface via the polyether block amide. As the silver compound is applied on the substrate surface on which the film has been formed, the silver in the silver compound may become bound to the methyl vinyl ether-maleic anhydride copolymer The silver that is bound in this manner exhibits antimicrobial effects Since the principal film component, i.e , the methyl vinyl ether-maleic anhydride copolymer, is firmly fixed to the substrate surface via polyether block amide, it can exhibit stable antimicrobial effects for a long time

After application of the silver compound, a liquid containing an antithrombotic substance may be applied on the surface of the above substrate on which the above film with bound silver has been formed The antithrombotic substance may thus also become bound to the above film. Because antithrombotic substances may become bound to the methyl vinyl ether-maleic anhydride copolymer, medical implements may be produced not only with antimicrobial properties but also with antithrombotic properties

In embodiments, an alkali treatment process may be performed by applying an aqueous alkaline solution on the surface of the substrate after the above film is formed By this alkali treatment process, the methyl vinyl ether-maleic anhydride copolymer incorporates alkali metals such as sodium and forms salts, manifesting lubricant properties when wet Medical implements that are additionally endowed with lubricant properties can thereby be manufactured.

### DETAILED DESCRIPTION

The medical implements of the present disclosure include substrates made of synthetic resins, having films that are formed on the surface of the substrates

Suitable synthetic resins that may form the substrate include, but are not limited to, polyurethanes, polyvinyl chlorides, nylons, nylon elastomers, combinations thereof, and the like In embodiments, polyurethane may be used as the substrate material because affinity with the polyether block amide described below may be favorable.

In embodiments, the films may include a methyl vinyl ether-maleic anhydride copolymer with bound silver as the main component The methyl vinyl ether-maleic anhydride copolymer that is the main component of the film may be an alternating copolymer of methyl vinyl ether and maleic anhydride. Silver may be bound to this methyl vinyl ether-maleic anhydride copolymer Without wishing to be bound by any theory, it is believed that the maleic anhydride portions of the methyl vinyl ether-maleic anhydride copolymer form carboxyl groups due to hydration, and the silver may be ionically bound to these carboxyl groups. Silver exhibits antimicrobial effects against a wide variety of bacteria, and if it is in an ionized state, it has greater antimicrobial effects. Since it is believed the carboxyl groups that are formed in the methyl vinyl ether-maleic anhydride copolymer are what bind antithrombotic substances or alkali metals such as sodium as described below, it may be desirable that the silver be partially bound, leaving room for the binding of these other substances

In embodiments, in addition to silver, antithrombotic substances may be bound to the methyl vinyl ether-maleic anhydride copolymer Antithrombotic substances are substances that inhibit formation of and solubilize thrombi that are generated by blood coagulation reactions If such antithrombotic substances are included in the film, they may be useful in preventing formation of blood clots when the medical implement is inserted or left in the body Suitable antithrombotic substances which may be used include, but are not limited to, urokinase, streptokinase, fibroplasminogen activator, plasmin, plinolase, heparin, hirudin, thrombomodulin, anti-platelet substances, combinations thereof, and the like In embodiments, it may be desirable to use urokinase as a thrombolytic enzyme because it can be covalently bound to carboxyl groups that are formed from the maleic anhydride portions of the methyl vinyl ether-maleic anhydride copolymer.

To endow the film with lubricant properties and antimicrobial properties, it may be desirable that the antithrombotic substance be partially bound to the above carboxyl groups

The medical implements of the present disclosure may be manufactured through any process within the purview of those skilled in the art In embodiments, a mixed solution including a solution with solubilized methyl vinyl ether-maleic anhydride copolymer and a solution with solubilized polyether block amide may be applied on the surface of a substrate made of synthetic resin to form a film. After formation of this film, a solution possessing a solubilized silver compound may be applied on the surface of the substrate on which the above film has been formed, and silver ions may be bound to the above film As used herein, "application" is used as a general term in which the mixed solution may be coated on the substrate surface. Application methods include various means such as spraying, dripping, immersion, combinations thereof, and the like Of these methods, application by immersion may be desirable because the methyl vinyl ether-maleic anhydride copolymer can be evenly and quickly fixed to appropriate sites on the substrate In embodiments, it may be desirable that the substrate surface be dried after immersion

The mixed solution that is applied on the substrate may include a solution in which methyl vinyl ether-maleic anhydride copolymer is solubilized Solvents for solubilization are not particularly limited as long as they are capable of solubilizing the copolymer. The above mixed solution also includes a solution in which polyether block amide is solubilized THF (tetrahydrofuran) may be utilized as a solvent The mixture of these solutions may be applied on the substrate surface Of the solubilized substances in the mixed solution, the polyether block amide is a block copolymer of polyether and polyamide Because its affinity with the substrate made of synthetic resin is high (affinity is particularly high when the substrate is polyurethane), it may be attracted to, and associates with, the substrate surface The polyether block amide also has an affinity for the methyl vinyl ether-maleic anhydride copolymer. For this reason, the methyl vinyl ether-maleic anhydride copolymer may be drawn to the substrate surface by an attractive force acting between the substances, with polyether block amide as the binder A film having methyl vinyl ether-maleic anhydride copolymer as the main component may thus be formed on the substrate surface by removing the various solvents solubilizing the various substances from the substrate surface. This film may be fixed firmly to this substrate surface with polyether block amide as binder.

A solution with a solubilized silver compound may then be applied on the substrate on which surface a film was formed as described above. By this application, silver ions in the silver compound may be ionically bound to carboxyl groups formed in the maleic anhydride portions of the methyl vinyl ether-maleic anhydride copolymer that is the main film component, and the silver may thus be incorporated into the film Antimicrobial properties are manifested by the silver that is incorporated in this manner Aqueous silver nitrate solutions, aqueous silver acetate solutions, aqueous silver perchlorate solutions, combinations thereof, and the like, may be used as aqueous silver compound solutions

A solution including antithrombotic substances can also be applied on the substrate surface The antithrombotic substances in the solution may be bound to carboxyl groups formed in the maleic anhydride portion of the methyl vinyl ether-maleic anhydride copolymer that is the main film component, and antithrombotic substances may thus be incorporated into the film. Antithrombotic properties are manifested by antithrombotic substances that are incorporated in this manner. Urokinase may be desirable as the antithrombotic substance In this case, urokinase may be covalently bound to maleic anhydride. On the other hand, silver may be bound ionically to maleic anhydride Since the binding strength of covalent bonds is stronger than that of ionic bonds, there are times when silver cannot be bound ionically if the antithrombotic substance is applied before the silver compound Meanwhile, if the silver compound is applied first and the antithrombotic substance is applied later, the urokinase with stronger binding strength can locally displace the silver and bind with the maleic anhydride even when the silver is bound on most of the maleic anhydride groups. Therefore, it may desirable to apply the antithrombotic substance after the silver compound

An alkali treatment process can be performed immediately after application of the film or after application of the silver compound In the alkali treatment process, an aqueous alkaline solution such as aqueous sodium hydroxide solution may be applied on the substrate surface on which the film is formed The carboxyl groups formed in the maleic anhydride portions of the film bind the sodium, and form salts Lubricant properties are thereby manifested on the substrate surface thus treated

The present disclosure thus encompasses methods for forming films having antimicrobial properties on the substrate surfaces of medical implements The method includes, in embodiments, fixing methyl vinyl ether-maleic anhydride copolymer to the surface of a synthetic resin medical implement substrate, and binding silver to the methyl vinyl ether-maleic anhydride copolymer fixed on or to the above substrate surface Furthermore, the present disclosure includes a film-forming method that includes a process wherein, after silver is bound to the methyl vinyl ether-maleic anhydride copolymer, antithrombotic substances may be bound to the methyl vinyl ether-maleic anhydride copolymer In other embodiments, the present disclosure includes a film-forming method that includes a process wherein, after the methyl vinyl ether-maleic anhydride copolymer of the substrate is fixed, the methyl vinyl ether-maleic anhydride copolymer may be alkali-treated.

The present disclosure also provides a method for forming films on the surface of medical implement substrates, wherein a mixed solution of a solution with solubilized methyl vinyl ether-maleic anhydride copolymer and a solution with solubilized polyether block amide is applied on the surface of a substrate made of synthetic resin, thereby forming a film on the surface of the substrate A solution possessing a solubilized silver compound may then be applied on the above substrate on which the above film is formed, with the silver becoming bound to the above film. The present disclosure also provides a film-forming method that includes a process wherein, after applying the solution with the solubilized silver compound in the above process, a solution including antithrombotic substances is applied. The present disclosure also provides a film-forming method including an alkali treatment process that is performed after the application of the film, in which an aqueous alkaline solution is applied on the surface of the above substrate on which the above film is formed

The following Examples are being submitted to illustrate embodiments of the present disclosure The Examples are intended to be illustrative only and are not intended to limit the scope of the present disclosure.

### EXAMPLE 1

About 2% acetone solution of methyl vinyl ether-maleic anhydride copolymer (Trade name: GANTREZ^{®} AN-169, made by ISP (International Specialty Products) referred to as 'A' below), and about 2% THF (tetrahydrofuran) solution of polyether block amide (Trade name: PEBAX^{®} 2533SA, made by Atochem Co , referred to as 'B' below) were mixed at a ratio of A:B of about 15:1 to make a coating mixture. Catheter tubes made of polyurethane having a 14 G diameter and 20 cm total length (Trade name: TECOFLEX^{®}, made by Thermedics Co) were prepared as the substrate and this substrate was immersed for several seconds in the above coating mixture After immersion, the substrate was withdrawn and dried under reduced pressure for about 3 hours at about 80°C. The substrate was then immersed in 0 1 N aqueous sodium hydroxide solution for about 3 minutes to accomplish alkali treatment.

After this, a substrate with formed film was immersed for about 24 hours at about 25°C in a 5% aqueous solution of silver nitrate (made by Nakaraitesc Co After immersion, the substrate was withdrawn, dried, and sterilized with ethylene oxide gas (EOG). A substrate with a formed film was manufactured in this manner.

### COMPARATIVE EXAMPLE 1

For Comparative Example 1, a film was formed on a substrate made of the same material as Example 1 above by the same process as set forth in Example 1 above After this, the same alkali treatment as in Example 1 was performed and then sterilization with EOG. A substrate with film formed on it was manufactured in this manner However, the substrate manufactured in this Comparative Example 1 was not subjected to submersion in aqueous silver nitrate, so it did not have silver bound in the film

Substrates with films formed according to the production methods described in Example 1 and Comparative Example 1 were cut into 1 cm lengths and made into samples to confirm whether they manifested antimicrobial effects by the inhibition circle test described below.

First, Staphylococcus aureus was prepared as a test microbe. The prepared bacteria were cultured for about 24 hours at about 37°C on soybean-casein-digest (SCD) agar medium (made by Nissui Seiyaku). After this, the cultured bacteria were suspended in physiological saline (sterilized) to an equivalent of about 10⁷ CFU/mL to prepare a suspension of bacterial cells (bacterial suspension).

Next, for inhibition circle-forming medium, SCD agar medium (made by Nissui Seiyaku) was steam-sterilized in an Erlenmeyer flask and then cooled to about 50°C in a hot bath After cooling, about 1/10 volume with respect to SCD agar medium of bacterial suspension was poured into the SCD agar medium, and agar medium comprising the bacterial strain (indicator bacterium-containing agar medium) was prepared

Next, the indicator bacterium-containing agar medium was poured into 8 cm diameter sterilized dishes and this agar medium was solidified in the dishes After solidification, holes of the same external diameter as the samples were made and samples were inserted in these holes. More indicator bacterium-containing medium was poured on top of these and solidified

Indicator bacterium-containing agar media inlaid with samples were prepared in this manner and were cultured for about 24 hours at about 37°C After culturing, the diameters of the inhibition circles formed were measured Measurement results are shown in Table 1.

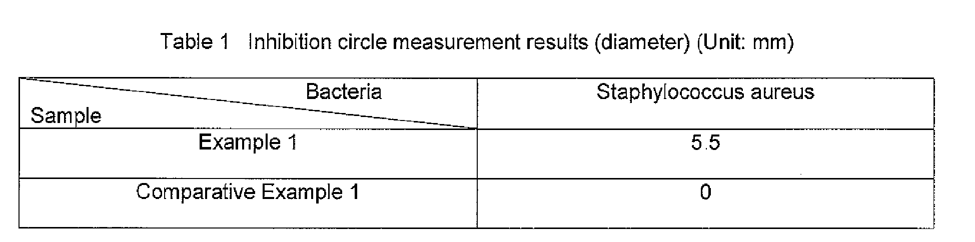

As can be seen from Table 1, an inhibition circle was formed in the agar medium inlaid with the sample prepared from Example 1 and thus Example 1 exhibited antimicrobial effects. In contrast to this, an inhibition circle was not formed on the agar medium inlaid with the sample prepared from Comparative Example 1 without adherent silver, and thus Comparative Example 1 did not generate antimicrobial effects. Therefore, it can be seen that silver was reliably incorporated into the sample by the method of Example 1, and the incorporated silver generated antimicrobial effects

For the sample prepared in Example 1, a film having methyl vinyl ether-maleic anhydride copolymer as the main component was formed on the substrate surface and lubricant properties were obtained by alkali treatment. Consequently, the samples prepared from Example 1 exhibited both antimicrobial and lubricant properties

### EXAMPLE 2

The 2% acetone solution of methyl vinyl ether-maleic anhydride copolymer of Example 1 (Trade name: GANTREZ^{®} AN-169, made by ISP, referred to as 'A' below) and the 2% THF solution of polyether block amide of Example 1 (Trade name: PEBAX^{®} 2533SA, made by Atochem Co , referred to as 'B' below) were mixed at a ratio of A:B of about 15:1 (referred to as 'A+B' below), tridodecyl methyl ammonium chloride (Trade name: Tridodecylmethylammonium chloride, made by Polysciences Co , referred to as 'C' below) was added at a ratio of A+B:C of about 5:1 to prepare a coating mixture with final proportions of A:B:C of about 3:2:1. Polyurethane catheter tubes of 14 G diameter and 20 cm total length (Trade name: TECOFLEX^{®}, made by Thermedics Co.) were prepared as substrate and this substrate was immersed for several seconds in the above coating mixture.. After immersion, the substrate was withdrawn and dried under reduced pressure for about 3 hours at about 80°C to form films on the substrate surface. A substrate with formed films was immersed for about 3 minutes in about 0.1 N aqueous sodium hydroxide solution and thus alkali-treated

After this, a substrate with a formed film was immersed for about 24 hours at about 25°C in about 5% aqueous silver nitrate solution (from Nakaraitesc Co) After immersion, it was withdrawn and dried

Next, a solution was prepared in which heparin sodium (made by Diosynth Co) was mixed to give a content of about 0 7% in acidic physiological saline having about 300 IU/mL urokinase (made by JCR Co) (pH about 4 6) and the substrate with a formed film was immersed in this solution for about 24 hours at about 5°C. After immersion, it was withdrawn and dried under reduced pressure Then, the substrate surface was sterilized by irradiating with a 40 kGy electron beam A substrate with a formed film was thus obtained.

ln this example, in addition to urokinase, heparin was bound to the substrate as an antithrombotic substance This heparin was bound to tridodecylmethylammonium chloride and the tridodecylmethylammonium chloride was bound to the polyether block amide by affinity bonds

### COMPARATIVE EXAMPLE 2

For Comparative Example 2, the same process as Example 2 above was performed to form a film on a substrate of the same material as Example 2. After this, the same alkali treatment as Example 2 above was performed and the same process of contacting the film and substrate with heparin sodium was also performed. After drying under reduced pressure, the substrate surface was sterilized by irradiating with a 40 kGy electron beam. A substrate with formed film was manufactured in this manner. The substrate manufactured in Comparative Example 2 differed in that it was not immersed in silver nitrate solution so that it did not possess bound silver.

### EXAMPLE 3

A substrate with a formed film was manufactured through the same processes as Example 2, except an 80% aqueous silver perchlorate solution was used instead of the 5% aqueous silver nitrate solution

### COMPARATIVE EXAMPLE 3

A substrate with a formed film was manufactured through the same processes as in Example 2, except a 10% aqueous silver carbonate solution was used instead of the 5% aqueous silver nitrate solution

Substrates with formed films manufactured in Example 2, Comparative Example 2, Example 3 and Comparative Example 3, were cut into 1-cm lengths and made into samples The same inhibition circle tests as were performed on devices of Example 1 and Comparative Example 1 above were performed on the respective samples to confirm whether antimicrobial effects were manifested Measurement results are shown in Table 2

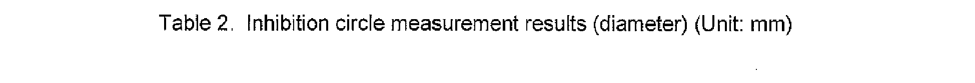

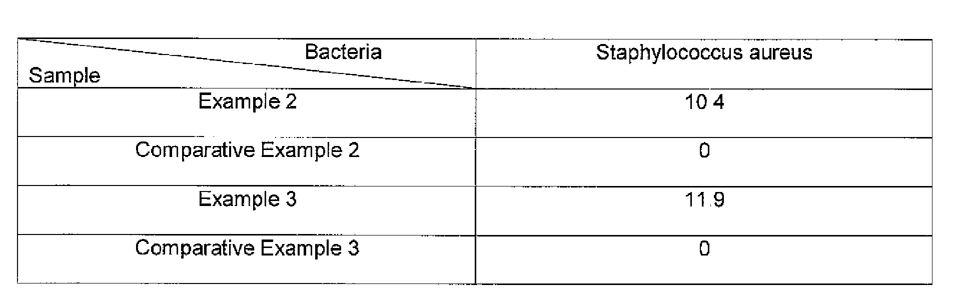

As can be seen from Table 2, samples prepared from Example 2 formed inhibition circles and antimicrobial effects were observed In contrast to this, inhibition circles were not formed with Comparative Example 2 without adherent silver, and thus antimicrobial effects were not generated. Consequently, it can be seen that silver was reliably incorporated in the substrate surface by the method of the present disclosure, and the incorporated silver generated antimicrobial effects

Samples prepared from Example 3 also formed inhibition circles. Therefore, it can be seen that, in addition to silver nitrate, when the silver compound was silver perchlorate, silver was effectively fixed as an antimicrobial agent in the film formed on the substrate surface Moreover, no inhibition circle was formed on samples made from Comparative Example 3 Therefore, it can be seen that when silver carbonate was used as the silver compound, silver was not fixed effectively in the film

Surface lubricant property tests were performed on all of the samples. This surface lubricant property test was performed based on feel when the surfaces of the various samples were touched with fingers Surface lubricant properties for the various samples were rated, with those that were felt to have good lubricant properties being rated as ⓪, those felt to have some lubricant properties as 'O,' and those felt to have scant lubricant properties as 'X' As a result of this, surface lubricant properties were ⓪ for all of the samples From these results, it was confirmed that the surface lubricant properties were good for all of the samples.

Antithrombotic property tests were performed on substrates (tubes) with formed films from Example 2, Example 3, Comparative Example 2, and Comparative Example 3. These antithrombotic property tests were performed by the Chandler loop method. Following the Chandler loop method, blood was added to fill the internal space of the substrate (tube) about halfway, the two ends were connected to each other to make a loop, this looped substrate (tube) was rotated, and the time from the beginning of substrate (tube) rotation until the blood lost fluidity and rotated with the substrate (tube) was measured If this time was long, antithrombotic properties were judged to be good, and if short, they were judged to be poor. Concrete details of this test are well-known. See, for example, Japanese Kokai Patent Application No. 2005-103238 for reference. As a result of such antithrombotic property tests, results were obtained demonstrating that antithrombotic properties were good for all of the substrates (tubes) in which the test was performed

Thus, it can be seen that in addition to antimicrobial properties, the medical tubes manufactured by Examples 2 and 3 were also equipped with surface lubricant properties and antithrombotic properties.

It will be appreciated that various of the above-disclosed and other features and functions, or alternatives thereof, may be desirably combined into many other different systems or applications Also that various presently unforeseen or unanticipated alternatives, modifications, variations or improvements therein may be subsequently may by those skilled in the art which are also intended to be encompassed by the following claims. Unless specifically recited in a claim, steps or components of claims should not be implied or imported from the specification or any other claims as to any particular order, number, position, size, shape, angle, color, or material

## Claims

1. A medical implement comprising:
a substrate comprising a synthetic resin;
a film comprising a methyl vinyl ether-maleic anhydride copolymer in combination with a polyether block amide fixed to at least a portion of a surface of the substrate; and
a silver ion bound to the methyl vinyl ether-maleic anhydride copolymer in combination with the polyether block amide

2. A medical implement as in claim 1, wherein the synthetic resin is selected from the group consisting of polyurethanes, polyvinyl chlorides, nylons, nylon elastomers, and combinations thereof

3. A medical implement as in claim 1, wherein the polyether block amide comprises a block copolymer of a polyether and a polyamide.

4. A medical implement as in claim 1, wherein the silver ion is derived from the group consisting of aqueous silver nitrate, aqueous silver acetate, aqueous silver perchlorate, and combinations thereof

5. A medical implement as in claim 1, wherein the silver ion is derived from silver nitrate

6. A medical implement as in claim 1, further comprising an antithrombotic substance

7. A medical implement as in claim 6, wherein the antithrombotic substance is selected from the group consisting of urokinase, streptokinase, fibroplasminogen activator, plasmin, plinolase, heparin, hirudin, thrombomodulin, anti-platelet substances, and combinations thereof

8. A medical implement as in claim 1, wherein the methyl vinyl ether-maleic anhydride copolymer comprises an alkali metal salt

9. A medical implement as in claim 8, wherein the alkali metal comprises sodium

10. A method comprising:
providing a medical implement comprising a substrate comprising a synthetic resin;
contacting at least a portion of a surface of the substrate with a solution comprising a solubilized methyl vinyl ether-maleic anhydride copolymer in combination with a polyether block amide;
forming a film that is fixed to the surface of the substrate; and
contacting the film with a silver ion that binds to the methyl vinyl ether-maleic anhydride copolymer in combination with the polyether block amide.

11. A method as in claim 10, wherein the silver ion is derived from the group consisting of aqueous silver nitrate, aqueous silver acetate, aqueous silver perchlorate, and combinations thereof.

12. A method as in claim 10, wherein the silver ion is derived from silver nitrate

13. A method as in claim 10, further comprising contacting the film with an antithrombotic substance.

14. A method as in claim 13, wherein the antithrombotic substance is selected from the group consisting of urokinase, streptokinase, fibroplasminogen activator, plasmin, plinolase, heparin, hirudin, thrombomodulin, anti-platelet substances, and combinations thereof.

15. A method as in claim 10, further comprising contacting the methyl vinyl ether-maleic anhydride copolymer with an aqueous alkali solution.

16. A method comprising:
providing a medical implement comprising a substrate comprising a synthetic resin;
contacting at least a portion of a surface of the substrate with a solution comprising a solubilized methyl vinyl ether-maleic anhydride copolymer in combination with a polyether block amide;
forming a film that is fixed to the surface of the substrate; and
contacting the film with a silver ion derived from the group consisting of aqueous silver nitrate, aqueous silver acetate, aqueous silver perchlorate, and combinations thereof

17. A method as in claim 16, wherein the silver ion is derived from silver nitrate.

18. A method as in claim 16, wherein the silver ion contacts the methyl vinyl ether-maleic anhydride copolymer

19. A method as in claim 16, further comprising contacting the film with an antithrombotic substance

20. A method as in claim 16, further comprising contacting the film with aqueous sodium hydroxide.
